# EUROPEAN PATENT SPECIFICATION

(11) **EP 2 032 170 B1**
(45) Date of publication and mention of the grant of the patent: **28.11.2012**
(21) Application number: 07747506.9
(22) Date of filing: 14.06.2007
(51) Int. Cl.: A61K 31/198, A61K 38/40, A23L 1/305, A61P 29/00

(54) **ANTI-INFLAMMATORY COMPOSITION COMPRISING GLYCINE AND LACTOFERRIN AND THE USE THEREOF**
ENTZÜNDUNGSHEMMENDE ZUSAMMENSETZUNG MIT GLYCIN UND LACTOFERRIN UND IHRE VERWENDUNG
COMPOSITION ANTI-INFLAMMATOIRE CONTENANT DE LA GLYCINE ET DE LA LACTOFERRINE ET UTILISATION DE CELLE-CI

(30) Priority: 14.06.2006 EP 06115496; 15.06.2006 US 814301 P
(43) Date of publication of application: 11.03.2009
(73) Proprietor: N.V. Nutricia, 2712 HM Zoetermeer (NL)
(72) Inventor: HARTOG, Anita, 6986 BK Angerlo (NL)
(74) Representative: Swinkels, Bart Willem
(86) International application number: PCT/NL2007/050283
(87) International publication number: WO 2007/145520

(56) References cited:
- EP-A2- 0 584 558
- WO-A-96/25861
- WO-A-03/099323
- WO-A2-2004/112697
- US-B1- 6 258 383
- C. GUILLEN, I.B. MCINNES, D. VAUGHAN, A.B.J. SPEEKENBRINK, J.H. BROCK: ARTHRITIS AND RHEUMATISM, vol. 43, no. 9, 2000, pages 2073-2080, XP009073763

## Description

### Field of the invention

The present invention relates to a nutritional or pharmaceutical composition with an anti-inflammatory activity suitable for patients with an inflammatory disease.

### Background of the invention

WO98/33509 describes the use of human lactoferrin for the treatment of inflammatory conditions.

WO03/099323 discloses the use of human lactoferrin for treating hyper-proliferative disease, i.e. amongst others rheumatoid arthritis and osteoarthritis.

EP0810829 discloses the enteral use of glycine (in combination with alanine and serine) for the diminution of tumor necrosis factor during inflammatory diseases such as rheumatoid arthritis and osteoarthritis.

WO 2004/112697 describes immune enhancing compositions comprising lactoferin.

It is an object of this invention to provide an improved alternative to anti-inflammatory nutritional compositions used to treat acute and chronic inflammatory diseases.

### Summary of the invention

The inventors surprisingly found that the combination of lactoferrin and glycine synergistically decrease inflammation *in vitro* and also *in vivo.* The synergistic effect is surprising because there are no indications in the literature suggesting that these two ingredients could affect each other's biological activity i.e. anti-inflammatory activity.

The invention can be used in enteral nutrition formulas and especially in clinical nutrition for patients with an inflammatory disease. These include, but are not limited to acute inflammation caused by a bacterial or viral infection e.g. meningitis, sepsis, malaria or chronic inflammatory diseases such as rheumatoid arthritis, osteoarthritis, psoriasis, chronic bronchitis, chronic obstructive pulmonary disease, inflammatory bowel disease (ulcerative colitis and crohn's disease), multiple sclerosis, etc.

### Detailed description of the invention

In accordance with the present invention, the compositions described and claimed herein will contain components suitable for inhibiting the inflammatory response in animals, including humans. In particular the invention concerns an anti-inflammatory composition comprising glycine and lactoferrin, wherein glycine is in the form of a free amino acid or in the form of a protein source consisting of at least 15wt% glycine based on weight of the total amino acid content of the composition or a combination thereof.

### Lactoferrin

Lactoferrin, or lactotransferrin, belongs to the transferrin family proteins, including serum transferrin, melanotransferrin, ovotransferrin etc., and is a globular multifunctional protein with antimicrobial activity. Transferrin family protein members are known to have similar effects. Hydrolysis of of lacto(trans)ferrin, or lactotransferrin proteolysis, produces lactoferricin, kaliocin-1 small peptides also with antimicrobial activity. Lacto(trans)ferrin is found in milk and many mucosal secretions such as tears and saliva. Lacto(trans)ferrin can be purified from milk or produced recombinantly. Human colostrum has the highest concentration, followed by human milk, then cow milk. For the purpose of the present invention lacto(trans)ferrin can be used in the form of a milk fraction enriched in lactoferrin wherein the milk is preferably originating from a mammal including, but not limited to, cow, goat or sheep. From an economical point of view the use of lactoferrin in the present invention is preferred especially bovine lactoferrin. However, in one embodiment lactoferrin may be wholly or partly replaced by another transferrin family protein. Also described are compositions wherein lactoferrin is partly or wholly replaced by serum transferrin, melanotransferrin and/or ovotransferrin, suitably by ovotransferrin. Lactoferrin peptides such as e.g. lactoferricin, can also be used and have the advantage that they are faster absorbed from the intestines in the blood. Preferably lactoferrin is used in a daily dose range between 4.0 mg and 2000 mg per day. Another preferred embodiment lies in the finding that the range wherein lactoferrin has an optimum of its beneficial anti-inflammatory effect is between 0.5 and 30 mg/kg body weight in a daily dose with a preferred range between 0.5 and 6 mg/kg body weight.

### Glycine

Glycine can be used as a free pure amino acid, enriched protein fractions or proteins rich in glycine. It will be appreciated that when glycine is used as a free amino acid it may be employed as non-toxic metal salts or acid addition salts. For example collagen is a protein rich in glycine (approximately one third of the amino acids in collagen is glycine, corresponding to approximately 15-20wt% glycine based on total amino acid content of protein). Hydrolysates of collagen are preferably used due to better product qualities such as solvability in water and taste. The dose is chosen from the range of 10 - 500 mg per kg bodyweight. The most preferred dose is within the range of 10 - 100 mg per kg bodyweight because the effectiveness may otherwise be less due to poor compliance that is expected in the higher doses.

Especially for use in the manufacture of a nutritional composition for the treatment of rheumatoid arthritis and osteoarthritis, collagen, gelatin or hydrolysates thereof may be used as a source of glycine because the other amino acids present in collagen and/or gelatin are also abundant in cartilage that needs to be replaced in arthritis patients. In general these protein sources have glycine content higher than 15 wt% of the total amino acid content of the proteins.

Thus a composition according to the invention is as follows: Anti-inflammatory composition comprising glycine and lactoferrin, wherein
a. glycine is present in a concentration of at least 40 mg per g protein based on weight of total protein of the composition, in the form of a free amino acid or in the form of a protein source consisting of at least 15 wt% glycine based on weight of the total amino acid content of the protein source or a combination thereof; and
b. lactoferrin is present in a concentration of 0.4 - 200 mg per gram protein
wherein preferably the the lactoferrin is of bovine origin.

### Poly unsaturated fatty acids (PUFA's)

Certain polyunsaturated fatty acids (PUFA's) are known anti-inflammatory compounds. These PUFA's can improve the anti-inflammatory effects of glycine and lactoferrin in a nutritional composition. Therefore a preferred embodiment comprises in addition of glycine and lactoferrin an effective amount of anti-inflammatory PUFA's. Preferably the fat comprises of DHA and/or EPA, preferably in an amount sufficient to induce anti-inflammatory effects. The present inventors found that polyunsaturated fatty acids, particularly eicosapentaenoic acid (EPA), docosahexaenoic acid (DHA) and gamma-linolenic acid (GLA), are capable of effectively improving the anti-inflammatory effects of glycine and lactoferrin. The fatty acids comprise at least one or more of the group: EPA, DHA and GLA. Omega-3 and omega-6 fatty acids are found in cold-water marine fish. Marine microorganisms are known to contain DHA, in particular Dinoglagellates. The preferred source for GLA is borage oil but also other sources such as linseed oil can be used. For the present invention it is not important which source of omega-3 fatty acids is used. Preferably at least 1.0 gram of EPA + DHA, is used as a daily dose in order to have a significant additional anti-inflammatory effect and preferably at least 2 g/day because this amount will give the best anti-inflammatory effect. The ratio n-3/n-6 in the product is preferably at least 0.5 and most preferred within 1-3.

### Use of the invention

The inventors found that a combination of glycine with lactoferrin provides an unexpectedly beneficial effect in reducing the negative effects of inflammatory processes as they occur in diseases that are characterized by an inflammatory component. These diseases include, but are not limited to diseases characterized by an acute inflammation caused by a bacterial or viral infection e.g. meningitis, sepsis, malaria or chronic inflammation such as rheumatoid arthritis, osteoarthritis, psoriasis, chronic bronchitis, chronic obstructive pulmonary disease, inflammatory bowel disease (ulcerative colitis and crohn's disease), multiple sclerosis, etc.

Anemia is a common co-morbidity in individuals with rheumatoid arthritis (RA). Estimates of the prevalence of mild anemia range between 30 and 60%. Increased production of inflammatory mediators in RA is linked to a cytokine-mediated decrease in erythropoietin response in the bone marrow, thereby leading to inadequate erythropoiesis. The anti-inflammatory effects of glycine and/or lactoferrin are therefore also beneficial for reducing the anemia often observed in patients with a chronic inflammatory disease. In some cases the addition of iron might improve the anemia as well. Lactoferrin is often prescribed as an efficient source of iron that is easily absorbed. The use of lactoferrin might therefore provide the patient with a double-edged sword, namely the anti-inflammatory effects as well as the easily absorbable iron in lactoferrin.

The composition according to the invention can successfully be used by patients with inflammatory diseases. Without being bound by theory, these patients often suffer from a form of cachexia induced by pro-inflammatory cytokines such as interleukin (IL)-1, IL-6 and TNF-α. By inhibiting the inflammatory response, positive effects on the cachexia symptoms could be anticipated. The symptoms of cachexia are caused by alterations in protein, fat, and carbohydrate metabolism including increased rate of glucose turnover, increased hepatic gluconeogenesis, glucose intolerance and elevated glucose levels. The weight loss often associated with cachexia is caused not only by a reduction in body fat stores but also by a reduction in total body protein mass, with extensive skeletal muscle wasting. Inhibition of cachexia is highly desirable because the inflammation-induced cachexia is associated with a high mortality and a low recovery rate of the patient suffering from inflammation. The anti-inflammatory effect of glycine with lactoferrin will improve the inflammation induced cachexia. Thus in one embodiment a composition according of the present invention is used for (the manufacture of a preparation for) the treatment of cachexia symptoms in patients with an inflammatory disease.

A composition according to the invention can be further be used for the manufacture of a medicament for the treatment of COPD, wherein the composition optionally further comprises one or more selected form the group consisting of coenzyme Q10, betaine, fish oil and tryptophan.

A composition according to the invention can further be used for the manufacture of a medicament for the treatment of inflammatory bowel diseases such as Crohns disease and Ulcerative colitis, wherein the composition optionally further comprises one or more selected from the group consisting of prebiotics, probiotics, colostrum and fish oil.

A composition according to the invention can further be used for the manufacture of a medicament for the treatment of Alzheimer, wherein the composition optionally further comprises one or more selected from the group consisting of UMP choline, phospholipids, fish oil, vitamin B1, vitamin B6 and vitamin B12. Alzheimer patients suffer from inflammation in the brain. Glycine and lactoferrin are both detectable in the brain indicating that these compounds are capable to pass the blood brain barrier. This makes the ingredients excellent compounds for the treatment of inflammation reactions in this protected environment.

Anti-inflammatory medication such as Non Steroid Anti-Inflammatory Drugs (NSAID's) often have severe detrimental inflammatory side-effects on the gastro-intestinal mucosa. This often results in peptic ulcers and duodenal ulcers caused by chronic use of these drugs. The inventors found that compositions according to the present invention can inhibit the formation of peptic ulcers induced by the NSAID indomethacin. A composition according to the invention comprising lactoferrin and glycine can beneficially be used for treatment and prevention of NSAID's -induced ulcers in the gastro-intestinal tract. In humans, the daily dose is at least 50 mg lactoferrin, preferably between 50 and 5000 mg and most preferred between 50 and 1000 mg lactoferrin. In certain circumstances the lactoferrin dose may be increased to twice this amount. This is dependent on the nutritional status of the individual patent. Poor iron status might also be a reason to increase the dose of lactoferrin.

### Nutritional compositions

In a preferred embodiment lactoferrin and glycine are present in a nutritional matrix comprising at least one selected from the group of ingredients consisting of: fat, carbohydrate, protein, vitamins, minerals and water. Additional nutritional ingredients can be added to enhance the anti-inflammatory effects in a specific target group. E.g. glucosamine and/or chondroitin can be added to a nutritional product for arthritis patients. Apart from its' *in vitro* anti-inflammatory effect, glucosamine is an important constituent of cartilage that helps to build new cartilage.

Another preferred embodiment comprises the use of branched chain amino acids. These amino acids play a crucial role in the management of muscle wasting that often co-occurs with inflammatory diseases. Therefore a nutritional composition according to the invention preferably is enriched in one or more of the branched chain amino acids leucin, isoleucine and valine. A protein source comprising 15-50 w% BCAA based on the total weight of amino acids present in the composition, preferably 20-40 w% and most preferred 25-35w% will give the optimal support for treatment and prevention of muscle wasting. The amino acids can be administered in the form of intact proteins, peptides, free amino acids or combinations thereof.

Another preferred embodiment comprises coenzyme Q10 and/or betaine. These two compounds have a beneficial effect on the capacity of muscle cells to store energy (in the form of glycogen). This helps to restore normal levels of activity in patients suffering from inflammation induced muscle wasting such as occurs in chronic obstructive pulmonary disease (COPD). Therefore a nutritional composition according to the invention preferably comprises coenzyme Q10 and/or betaine. The preferred dose of betaine is between 50 and 50000 mg per day more preferably between 100 and 10000 mg per day and most preferred between 1000 and 10000 mg per day. The preferred dose of Q10 is in the range of 10 to 2000 mg/day and more preferably 20 to 1000 mg/day and most preferably between 90 to 390 mg/day.

Another preferred embodiments comprises apart from lactoferrin and glycine according to the invention, prebiotics and/or probiotics for the manufacture of a nutritional composition for the treatment and/or prevention of inflammatory bowel diseases. Preferably the composition further comprises an EPA/DHA containing oil blend. The prebiotics are preferably selected from the group consisting of: Galacto oligosaccharides, fructo oligosaccharides, xylo oligosaccharides, pectins, xanthan gum, galactomannans, and glucomannans.

The nutritional compositions can have different forms such as the form of a bar, powder, liquid sip feed, thickened drink, tube feed, etc. Especially tube feed needs mentioning because the target group often lacks appetite. The liquid nutrition that can be used as a tube feed should preferably have a viscosity between 1-20 Pa.s. Tube feed should preferably deliver between 0.5 and 1.5 kcal/ml. Frequently a nutritional matrix will contain vitamins, minerals, trace minerals and the like to provide balanced nutrition. Preferably these ingredients are present in amounts according to the 'Foods for Special Medical Purposes' (FSMP) regulations. However due to high oxidative stress in the inflamed tissues additional antioxidant compounds preferably are used. Antioxidants like N-acetyl cysteine could be used to improve the anti-oxidative status of the patients with inflammatory diseases.

### Concentrations:

Although concentrations are not part of the invention; certain limits in concentrations are applicable. In a liquid composition Lactoferrin preferably is present in the range of 0.1 - 25 mg/ml, preferably for the best compliance the concentration is higher, in the range of 0.5 -10 mg/ ml. Glycine is preferably present in the range of 0.5 - 50 mg/ml. When PUFA's are present the concentration range preferably is 2 - 30 mg PUFA, preferably 2-20 mg EPA+DHA per ml.

In complete nutritional products according to this invention, the protein content is in a range from 8-60 en% or for example 8-45 en%, preferably between 10-35 en% and further comprising 10-30 en% fat, 10-70 en% carbohydrates and at least 2.5 gram per 100 gram dry weight of the total product of a mixture of vitamins and minerals comprising at least one chosen from vitamin D, vitamin E, vitamin B6, folic acid, CoQ10, betaine, calcium and selenium.

Protein is defined as proteinaceous material essentially consisting of amino acids as is indicated on the product label. E.g. Milk, whey, casein, other animal and plant proteins and hydrolysates thereof, but also free amino acids and their salts fall within the definition of protein.

The following examples are in support of the invention that glycine and lactoferrin in combination synergistically suppress inflammatory response. Some examples are given of nutritional compositions for different inflammatory diseases. They all comprise glycine and lactoferrin, but specific other ingredients are added according to specific additional needs of the indicated sub-groups of inflammatory deceases.

### Example 1. Synergy of lactoferrin and glycine in an inflammatory mouse model

### Materials and methods:

Balb/C mice (8 weeks of age, Charles River, Maastricht, The Netherlands) were randomly assigned two control and three test groups and supplemented daily for three days with 0.1 mg lactoferrin (DMV, Veghel, The Netherlands), 50 mg glycine (Sigma, Zwijndrecht, The Netherlands) or a combination of both components (test groups 3, 4 and 5 respectively) or vehicle (control groups 1 and 2) was administered by gavage. At day two of the supplementation ear thickness was measured using a micrometer (Mitutoyo Digimatic, Veenendaal, The Netherlands) Subsequently 25 µl 0.5% zymosan (Sigma) in PBS (group 2-5) or vehicle (group1) was injected subcutaneously in both ears. Ear thickness was measured 3, 6 and 24 hours (example 1) after injection after which the animals were sacrificed.

The spleen was removed and pushed through a cell strainer. Red blood cell lysis was performed in 5 ml of could lysis buffer (4.15 g NH₄Cl, 0.5 g KHCO₃ and 18.6 mg Na₂EDTA in 1 liter water at pH 7,3) for 5 minutes. Cells were washed twice in could RPMI (Invitrogen, Merelbeke, Belgium) and after centrifugation diluted to a concentration of 1.10⁷ cells/ml. TNF-α producing cells were detected by ELISpot assay in the absence or presence of 1 µg/ml lipopolysaccharide (Sigma) (example 2).

In mice injected in the ear with zymosan, lactoferrin (LF) and glycine (Gly) supplemented mice show a highest decrease in ear swelling when compared to non-supplemented mice, see Table 1. A clear synergistic effect of LF and Gly is observed. At time point 6 hrs the combination of gly and LF completely inhibited the increase in ear swelling which is statistically significant different from the groups supplemented with the individual ingredients LF and Gly.

| | **DECREASE IN EAR SWELLING** | | |
|---|---|---|---|
| **Time (hrs)** | **Lactoferrin** | **Glycine** | **Glycine+lactoferrin** |
| **3** | 39±12 | 10±16 | 63±22 |
| **6** | 53±17 | 28±20 | 111±14^{#} |

| | | | |
|---|---|---|---|
| # Maximal effect, no increase in ear swelling | | | |

### Example 2. Additive effect of LF and Glycine on ex-vivo inflammatory marker TNF

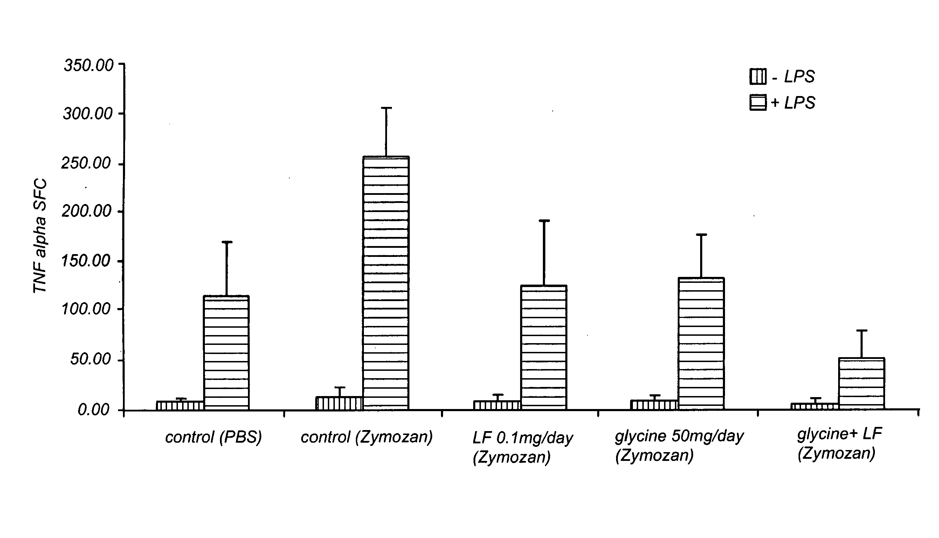

Spleen cells were collected from mice with a zymosan-induced inflammation in the ear after three days of oral supplementation with water (=control), lactoferrin (LF), glycine or a combination of LF and glycine. The number of TNF-α producing cells was determined using an ELISpot assay. The results are shown in figure 1. LF and glycine inhibit the LPS stimulated number of TNF producing cells by 92% and 97% respectively (back to not zymosan treated control levels). The combination of glycine and lactoferrin inhibited the number of TNF-α positive cells 50% below the not PBS injected control. This unexpected high rate of inhibition makes the combination of glycine with lactoferrin an excellent choice for the treatment of inflammatory diseases.

### Example 3. Dose response of lactoferrin in an inflammatory mouse model

### Materials and methods:

Balb/C mice (8 weeks of age) were randomly assigned two controls and three test groups and supplemented daily for three days with 0.1 mg, 1 mg or 5 mg lactoferrin (test groups 3, 4 and 5 respectively) or vehicle (control groups 1 and 2) was administered by gavage. At day two of the supplementation ear thickness was measured using a micrometer. Subsequently 25 µl 0.5% zymosan in PBS (group 2-5) or vehicle (group1) was injected subcutaneously in both ears. Ear thickness was measured 3, 6 and 24 hours (example 3) after injection after which the animals were sacrificed.

### Dose response of lactoferrin in an inflammatory mouse model

| | time 1 hr | | time 2 hr | | time 4 hr | |
|---|---|---|---|---|---|---|
| Group | swelling | sem | swelling | sem | swelling | sem |
| Control | 24 | 4.9 | 47 | 5 | 60 | 8 |
| Zymosan | 111 | 4.0 | 166 | 4 | 192 | 10 |
| 0.1 mg LF | 78 | 7.0 | 108 | 5 | 109 | 10 |
| 1mg LF | 73 | 10.5 | 105 | 15 | 121 | 14 |
| 5mg LF | 99 | 5 | 153 | 16 | 204 | 18 |

The optimal dose is higher than 0 and less than 5 mg lactoferrin. 5 mg lactoferrin clearly inhibits the zymosan induced swelling less than a dose of 0.1 and 1.0 mg lactoferrin indicating the optimal dose of lactoferrin in combination with glycine will have a similar optimum.

### Example 4 Nutritional liquid composition.

Per 100 ml.

| 7.6 g protein | |
|---|---|
| | 3.0 g casein |
| | 3.0 g whey |
| | 50 mg Lacoferrin |
| | + 2.0 g glycine as free amino acid |
| | |

| 22.5 g carbohydrates | |
|---|---|
| | glucose |
| | lactose |
| | maltose |
| | scharose |
| | polysaccharides |
| | others |
| | |

| 3.3 g fat | |
|---|---|
| | saturated fat |
| | MUFA |
| | PUFA |
| Minerals according to FSMP regulations | |
| Vitamins according to FSMP regulations | |
| Osmolarity between 400 - 800 mOsmol/L | |

### Example 5. Nutritional composition for arthritis patients comprising LF+Gly

Per 100 kcal

| 9 g Protein | |
|---|---|
| | 2.7 g casein |
| | 5 g collagen hydrolysate (1.0 g added glycine) |
| | 1.0 - 2.0 g glycine added amino acid |
| | 0.050-0.1 g lactoferrin |
| | |

| 7.5 g carbohydrates | |
|---|---|
| | |

| 3.9 g fat | |
|---|---|
| | 2 g EPA/DHA oil |
| | 1.9 g other oil |
| | |
| Minerals and vitamins according to FSMP standard + 0.5 - 5 gram Glucosamine + 0 - 5 gram Chondroitine + Calcium + Vitamin D | |

**Example 6.** Nutritional composition for COPD comprising LF+gly

| 5 gram protein | |
|---|---|
| | 1.8 g casein |
| | 1.8 g whey |
| | 1.3 g added glycine |
| | 0.05 -5 g added tryptophan |
| | |

| 15 g carbohydrates | |
|---|---|
| | |

| 2.2 g fat | |
|---|---|
| | 1.5 g EPA/DHA oil |
| | 0.7 g other fat |
| 90 mgQ10 | |
| 750 mg betaine | |

### Example 7. Nutritional composition for IBD comprising LF+Gly

Per 150 kcal

| 6 g protein | |
|---|---|
| | 1.1 g casein |
| | 3.3 g whey |
| | 1.2 g glycine |
| | 150 mg Lactoferrin |
| | |

| 18.7 g carbohydrates | |
|---|---|
| | |

| 5.8 g fat | |
|---|---|
| | 2 g EPA/DHA oil |
| | 3.8 g other oil |

| other ingredients that may be present: | |
|---|---|
| +Prebiotics (e.g. Galacto oligosaccharides, fructo oligo and/or poly saccharides) | |
| +probiotics (e.g. lactobacilli or bifidobacteria) | |

### Example 8. Nutritional composition for Alzheimer comprising LF+Gly

Per 100ml

| | |
|---|---|
| UMP | 0.05 -2.0 gram |
| Choline | 0.1 -2.0 gram |
| fish oil | 0.5 - 8 gram |
| phospholipids | 0.05 - 2.0 gram |
| at least two of Vitamin B11, B6, B12 in the ranges of | |
| B11 | 0.1-0.5 mg |
| B6 | 0.5-2.0 mg |
| B12 | 0.5-100 µg |

## Claims

1. Anti-inflammatory nutritional composition comprising glycine and lactoferrin, wherein
a. glycine is present in a concentration of at least 40 mg per g protein based on weight of total protein of the composition, in the form of a free amino acid or in the form of a protein source consisting of at least 15 wt% glycine based on weight of the total amino acid content of the protein source or a combination thereof; and
b. lactoferrin is present in a concentration of 0.4- 200 mg per gram protein.

2. Composition according to claim 1 wherein the lactoferrin is bovine lactoferrin.

3. Composition according to any of claims 1 or 2 wherein the composition further comprises polyunsaturated fatty acids.

4. Composition according to claim 3 wherein the ratio n-3/n-6 of the polyunsaturated fatty acids in the product is at least 1.

5. Composition according to any of claims 1-4, wherein the protein source is selected from the group consisting of collagen, gelatin or hydrolysates thereof.

6. Liquid composition according to any of claims 1-5 wherein the concentration of glycine is in the range of 0.5 - 50 mg/ml.

7. Liquid composition according to claim 6 wherein the concentration lactoferrin is in the range of 0.1 - 25 mg/ml.

8. Nutritional composition according to any of claims 1-7, comprising 8-60 en% protein, 10-30 en% fat, 10-70 en% carbohydrates and at least 2.5 gram per 100 gram dry weight of the total product of a mixture of vitamins and minerals comprising at least one chosen from vitamin D, vitamin E, vitamin B6, folic acid, CoQ10, betaine, calcium and selenium.

9. A composition according to any of the claims 1-8 for use in the the treatment and prevention of inflammatory diseases.

10. A composition according to any of the claims 1-8 for use in the treatment of cachexia symptoms in patients with an inflammatory disease.

11. Composition according to claim 9, wherein the inflammatory disease is arthritis.

12. Composition for use according to any of claims 9-11 wherein the daily dose of glycine is in the range of 10 - 500 mg per kg bodyweight.

13. Composition for use according to any of claims 9-12 wherein the daily dose of lactoferrin is in the range of 0.5 to 30 mg per kg body weight.

14. A composition according to any of the claims 1-8 for use in the treatment of arthritis patients, wherein the composition optionally further comprises one or more selected form the group consisting of tryptophan, glucosamine, chondroitine, EPA containing oil, calcium and vitamin D.

15. A composition according to any of the claims 1-8 for use in the treatment of COPD, wherein the composition optionally further comprises one or more selected form the group consisting of coenzyme Q10, betaine, fish oil and tryptophan.

16. A composition according to any of the claims 1-8 for use in the treatment of intestinal inflammatory diseases, wherein the composition optionally further comprises one or more selected from the group consisting of prebiotics, probiotics, colostrum and fish oil.

## Patentansprüche

1. Entzündungshemmende Nährstoffzusammensetzung, umfassend Glycin und Lactoferrin, wobei
a. Glycin in einer Konzentration von wenigstens 40 mg pro g Protein, bezogen auf das Gewicht des Gesamtproteins der Zusammensetzung, in Form einer freien Aminosäure oder in Form einer Proteinquelle, bestehend aus wenigstens 15 Gew.-% Glycin, bezogen auf das Gewicht des gesamten Aminosäuregehalts der Proteinquelle, oder einer Kombination davon, vorhanden ist; und
b. Lactoferrin in einer Konzentration von 0,4-200 mg pro Gramm Protein vorhanden ist.

2. Zusammensetzung nach Anspruch 1, wobei das Lactoferrin Rinderlactoferrin ist.

3. Zusammensetzung nach einem der Ansprüche 1 oder 2, wobei die Zusammensetzung außerdem mehrfach ungesättigte Fettsäuren umfasst.

4. Zusammensetzung nach Anspruch 3, wobei das Verhältnis n-3/n-6 der mehrfach ungesättigten Fettsäuren in dem Produkt wenigstens 1 beträgt.

5. Zusammensetzung nach einem der Ansprüche 1-4, wobei die Proteinquelle ausgewählt ist aus der Gruppe bestehend aus Kollagen, Gelatine oder Hydrolysaten davon.

6. Flüssige Zusammensetzung nach einem der Ansprüche 1-5, wobei die Konzentration von Glycin im Bereich von 0,5-50 mg/ml liegt.

7. Flüssige Zusammensetzung nach Anspruch 6, wobei die Konzentration von Lactoferrin im Bereich von 0,1-25 mg/ml liegt.

8. Nährstoffzusammensetzung nach einem der Ansprüche 1-7, umfassend 8-60 en% Protein, 10-30 en% Fett, 10-70 en% Kohlenhydrate und wenigstens 2,5 Gramm pro 100 Gramm Trockengewicht des Gesamtprodukts von einer Mischung von Vitaminen und Mineralien, umfassend wenigstens eines ausgewählt aus Vitamin D, Vitamin E, Vitamin B6, Folsäure, CoQ10, Betain, Calcium und Selen.

9. Zusammensetzung nach einem der Ansprüche 1-8 zur Verwendung bei der Behandlung und Verhütung von entzündlichen Erkrankungen.

10. Zusammensetzung nach einem der Ansprüche 1-8 zur Verwendung bei der Behandlung von Kachexie-Symptomen bei Patienten mit einer entzündlichen Erkrankung.

11. Zusammensetzung nach Anspruch 9, wobei die entzündliche Erkrankung Arthritis ist.

12. Zusammensetzung zur Verwendung nach einem der Ansprüche 9-11, wobei die Tagesdosis von Glycin im Bereich von 10-500 mg pro kg Körpergewicht liegt.

13. Zusammensetzung zur Verwendung nach einem der Ansprüche 9-12, wobei die Tagesdosis von Lactoferrin im Bereich von 0,5 bis 30 mg pro kg Körpergewicht liegt.

14. Zusammensetzung nach einem der Ansprüche 1-8 zur Verwendung bei der Behandlung von Arthritispatienten, wobei die Zusammensetzung gegebenenfalls außerdem eines oder mehrere, ausgewählt aus der Gruppe bestehend aus Tryptophan, Glucosamin, Chondroitin, EPA enthaltendem Öl, Calcium und Vitamin D, umfasst.

15. Zusammensetzung nach einem der Ansprüche 1-8 zur Verwendung bei der Behandlung von COPD, wobei die Zusammensetzung gegebenenfalls außerdem eines oder mehrere, ausgewählt aus der Gruppe bestehend aus Coenzym Q10, Betain, Fischöl und Tryptophan, umfasst.

16. Zusammensetzung nach einem der Ansprüche 1-8 zur Verwendung bei der Behandlung von entzündlichen Darmerkrankungen, wobei die Zusammensetzung gegebenenfalls außerdem eines oder mehrere, ausgewählt aus der Gruppe bestehend aus Präbiotika, Probiotika, Kolostrum und Fischöl, umfasst.

## Revendications

1. Composition nutritionnelle anti-inflammatoire comprenant de la glycine et de la lactoferrine, où
a. la glycine est présente en une concentration d'au moins 40 mg par g de protéines sur la base du poids de protéines totales de la composition, sous forme d'un aminoacide libre ou sous forme d'une source de protéines consistant en au moins 15 % en poids de glycine sur la base du poids de la teneur en aminoacides totale de la source de protéines ou d'une combinaison de ceux- ci ; et
b. la lactoferrine est présente en une concentration de 0,4 - 200 mg par gramme de protéines.

2. Composition selon la revendication 1 où la lactoferrine est la lactoferrine bovine.

3. Composition selon l'une quelconque des revendications 1 et 2 où la composition comprend en outre des acides gras polyinsaturés.

4. Composition selon la revendication 3 où le rapport n-3/n-6 des acides gras polyinsaturés dans le produit est au moins 1.

5. Composition selon l'une quelconque des revendications 1-4 où la source de protéines est choisie dans le groupe consistant en le collagène, la gélatine et leurs hydrolysats.

6. Composition liquide selon l'une quelconque des revendications 1-5 où la concentration de glycine est dans la plage de 0,5 - 50 mg/ml.

7. Composition liquide selon la revendication 6 où la concentration de lactoferrine est dans la plage de 0,1 - 25 mg/ml.

8. Composition nutritionnelle selon l'une quelconque des revendications 1-7 comprenant 8-60 en % de protéines, 10-30 en % de graisses, 10-70 en % de glucides et au moins 2,5 grammes pour 100 grammes de poids sec du produit total d'un mélange de vitamines et de minéraux comprenant au moins un choisi parmi la vitamine D, la vitamine E, la vitamine B6, l'acide folique, CoQ10, la bétaïne, le calcium et le sélénium.

9. Composition selon l'une quelconque des revendications 1-8 destinée à être utilisée dans le traitement et la prévention des maladies inflammatoires.

10. Composition selon l'une quelconque des revendications 1-8 destinée à être utilisée dans le traitement des symptômes de cachexie chez des patients ayant une maladie inflammatoire.

11. Composition selon la revendication 9 où la maladie inflammatoire est l'arthrite.

12. Composition destinée à être utilisée selon l'une quelconque des revendications 9-11 où la dose journalière de glycine est dans la plage de 10-500 mg par kg de poids corporel.

13. Composition destinée à être utilisée selon l'une quelconque des revendications 9-12 où la dose journalière de lactoferrine est dans la plage de 0,5 à 30 mg par kg de poids corporel.

14. Composition selon l'une quelconque des revendications 1-8 destinée à être utilisée dans le traitement des patients arthritiques où la composition comprend éventuellement en outre un ou plusieurs choisis dans le groupe consistant en tryptophane, glucosamine, chondroïtine, huile contenant de l'EPA, calcium et vitamine D.

15. Composition selon l'une quelconque des revendications 1-8 destinée à être utilisée dans le traitement de la COPD où la composition comprend éventuellement en outre un ou plusieurs choisis dans le groupe consistant en coenzyme Q10, bétaïne, huile de poisson et tryptophane.

16. Composition selon l'une quelconque des revendications 1-8 destinée à être utilisée dans le traitement des maladies inflammatoires intestinales où la composition comprend éventuellement en outre un ou plusieurs choisis dans le groupe consistant en les prébiotiques, les probiotiques, le colostrum et l'huile de poisson.
